(19) Europäisches Patentamt
European Patent Office
Office européen des brevets

(11) **EP 4 721 674 A1**

(12) **EUROPEAN PATENT APPLICATION**

(43) Date of publication:
08.04.2026 Bulletin 2026/15

(21) Application number: 24204126.7

(22) Date of filing: 02.10.2024

(51) International Patent Classification (IPC):
*A61B 8/04* (2006.01)   *A61B 8/06* (2006.01)
*A61B 8/08* (2006.01)   *A61B 8/00* (2006.01)
*A61B 8/02* (2006.01)

(52) Cooperative Patent Classification (CPC):
A61B 8/04; A61B 8/065; A61B 8/0883;
A61B 8/0891; A61B 8/488; A61B 8/5223;
A61B 8/02

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Koninklijke Philips N.V.
5656 AG Eindhoven (NL)

(72) Inventors:
• LAU, Kevin Daniel Seng Hung
5656 AG Eindhoven (NL)
• JOSHI, Rohan
5656 AG Eindhoven (NL)
• DINIS FERNANDES-KUILBOER, Catarina
5656 AG Eindhoven (NL)

(74) Representative: Philips Intellectual Property &
Standards
High Tech Campus 52
5656 AG Eindhoven (NL)

(54) **HEMODYNAMIC PARAMETER ESTIMATION**

(57) A method for non-invasive hemodynamic monitoring of patients. The method comprises using ultrasound to obtain a continuous blood velocity signal and continuous arterial diameter signal. The method further comprises extracting waveform features from these two signals which can be used to estimate hemodynamic parameters. A transfer function is used to convert values of a pre-defined set of waveform features into values of one or more hemodynamic parameters. To enhance accuracy, ultrasound data is acquired over a time window and an ensemble average blood velocity waveform is computed from a series of waveforms detectable in the blood velocity-time signal over said time window.

FIG. 2

EP 4 721 674 A1

**Description**

FIELD OF THE INVENTION

The present invention relates to hemodynamic parameter estimation using ultrasound data of a blood vessel.

BACKGROUND OF THE INVENTION

Hemodynamic monitoring is an important aspect of patient monitoring.

Hemodynamics refers to the physical principles governing the circulation of blood in the cardiovascular system. It involves inter alia the assessment of blood volume, pressure, and the oxygenation level in the blood. Monitoring these parameters is of significance in the management of patients in intensive care units (ICUs) and during high-risk surgeries.

Hemodynamic monitoring in the current state of the art involves invasive monitoring methods such as an arterial catheter. Non-invasive methods for monitoring hemodynamic status would be of value.

It would be of advantage to identify a non-invasive hemodynamic monitoring method with higher accuracy than existing methods.

SUMMARY OF THE INVENTION

The invention is defined by the claims.

According to examples in accordance with an aspect of the invention, there is provided a computer-implemented method for deriving one or more hemodynamic parameters of a subject. The method comprises receiving ultrasound data from a blood vessel of the subject, wherein said ultrasound data includes B-Mode data and pulsed wave Doppler data. The method further comprises deriving from the received ultrasound data a blood velocity-time signal representative of blood velocity at a measurement location of the blood vessel over a time window. The method further comprises deriving from the received ultrasound data an arterial diameter signal representative of a diameter of said at least one blood vessel, or a parameter proportional thereto, at the measurement location over the time window. The method further comprises computing an ensemble average blood velocity waveform from a plurality of blood velocity waveforms present in the blood velocity-time signal. Each of the plurality of blood velocity waveforms corresponds to a single heartbeat. The method further comprises determining a first set of parameters, where this includes: determining from the ensemble average blood velocity waveform one or more pre-defined blood velocity waveform features, and determining from the arterial diameter signal one or more pre-defined arterial diameter waveform features. The method further comprises retrieving a pre-defined transfer function configured to receive said first set of parameters as inputs and to generate the one or more hemodynamic parameters as output. The method further comprises processing the first set of parameters with the transfer function to derive values for the one or more hemodynamic parameters.

Embodiments of the invention provide an improved hemodynamic parameter transfer function in which features are extracted from an ensemble average waveform computed from multiple waveforms acquired over a complete measurement, rather than or in addition to extracting features from individual waveforms in the signal. This improves robustness of the method.

In some embodiments, the one or more pre-defined blood velocity waveform features determined from the ensemble average blood velocity waveform include: an acceleration parameter indicative of a steepness of a leading edge of the ensemble average blood velocity waveform.

In some embodiments, the one or more pre-defined blood velocity waveform features determined from the ensemble average blood velocity waveform include: a deceleration parameter indicative of a steepness of a trailing edge of the ensemble average blood velocity waveform.

In some embodiments, the one or more pre-defined blood velocity waveform features determined from the ensemble average blood velocity waveform include: a velocity full-width-half-maximum (FWHM) parameter indicative of a full-width-half-maximum of the ensemble average blood velocity waveform.

In some embodiments, the method further comprises obtaining a blood pressure waveform signal for the subject indicative of a blood pressure waveform for the subject over the time window. In some embodiments, the computing the first set of parameters further includes: extracting from the blood pressure waveform signal one or more pre-defined blood pressure waveform features.

In some embodiments, the method further comprises: computing from the blood pressure waveform signal an ensemble average blood pressure waveform from a plurality of blood pressure waveforms present in the blood pressure waveform signal. In some embodiments, the determining the first set of parameters further includes deriving from the ensemble average blood pressure waveform one or more predefined blood pressure waveform features. Each of the plurality of blood pressure waveforms may correspond to a single heartbeat. In other words, each of the plurality of blood pressure waveforms may represent arterial blood pressure as a function of time over the course of a single heartbeat.

In some embodiments, the one or more predefined blood pressure waveform features include a blood pressure acceleration parameter indicative of a rise time between a foot of the ensemble average blood pressure waveform and a peak of the ensemble average blood pressure waveform.

In some embodiments, the computing the first set of parameters further includes: computing from the blood velocity-time signal one or more further pre-defined blood velocity waveform features.

In some embodiments, the one or more further pre-defined blood velocity waveform features comprise at least one of: an interdecile range of the velocity-time signal over the time window; a mean value of blood velocity over the time window; a mean value of peak systolic velocity over the time window; a mean value of the blood velocity over the time window, normalized by a number of heart cycles spanned by the time window; and an integral of the velocity-time signal with respect to time over the time window, normalized by the number of heart cycles spanned by the time window.

In some embodiments, the one or more arterial diameter waveform features comprise at least one of: a mean value of the arterial diameter over the time window; and a mean value of a cross-sectional area of the at least one blood vessel over the time window.

In some embodiments, the computing from the arterial diameter signal one or more pre-defined arterial diameter waveform features comprises: computing an ensemble average arterial diameter pulse waveform from a plurality of arterial diameter pulse waveforms present in the arterial diameter signal; and computing from the ensemble average arterial diameter waveform an arterial diameter acceleration parameter indicative of a rise time between a foot of the ensemble average arterial diameter waveform and a peak of the ensemble average arterial diameter waveform.

In some embodiments, the first set of parameters may further include any one or more of: skewness of the ensemble average velocity waveform, kurtosis of the ensemble average velocity waveform, Full Width Half Maximum (FWHM) of the ensemble average velocity waveform, and an acceleration of the ensemble average velocity waveform indicative of a steepness of a leading edge of the ensemble average velocity waveform.

"Skewness" refers to a measure of the asymmetry of the probability distribution of a real-valued random variable about its mean. "Kurtosis" refers to a measure of the "tailedness" of the probability distribution of a real-valued random variable. In mathematics, skewness is sometimes understood as the third standardized moment of the probability distribution, and kurtosis may be understood as the fourth standardized moment of the probability distribution. "Full Width Half Maximum (FWHM)" is a measure of the width of a pulse at half of its maximum intensity.

In some embodiments, the first set of parameters may include a value for an area under a pressure-volume loop, the pressure-volume loop computed using the ensemble average velocity waveform, the ensemble average blood pressure waveform and the arterial diameter signal.

In some embodiments, the first set of parameters may include a value for a steepness of the rising edge (or upslope) of a pressure-volume loop, the pressure volume loop computed using the ensemble average velocity waveform, the ensemble average arterial pressure waveform and the arterial diameter signal.

In some embodiments, the first set of parameters may include values for one or more waveform features extracted from the velocity-time signal, for example any one or more of: peak systolic velocity, end diastolic velocity, area under the velocity-time signal per heartbeat, mean velocity, mean velocity per heartbeat, mean square velocity, and inter-decile range of the velocity-time signal.

The area under the velocity-time signal per heartbeat can be calculated as the integral of the velocity-time signal over the relevant time interval across which the signal spans, divided by the number of heartbeats which are present in that time interval.

The mean velocity per heartbeat can be calculated as the mean of the velocity-time signal samples over the relevant time interval across which the signal spans, divided by the number of the number of heartbeats which are present in that time interval.

In some embodiments, the first set of parameters may include values for each of one or more features which are computed from a combination of the velocity-time signal and the arterial diameter signal, from a combination of the velocity-time signal and the diameter signal, and/or from a combination of the arterial diameter signal and the blood pressure waveform signal.

For example, in some embodiments, the first set of parameters may include a value for a square of the difference between squared values of peak systolic velocity (PSV) and end diastolic velocity (EDV) divided by squared value of diameter squared. This is referred to by the label 'deltaVelByDia' in the Detailed Description section of this document.

By way of further example, in some embodiments, the first set of parameters may include a value for mean blood pressure squared divided by a value of peak systolic velocity (PSV). The value for the mean blood pressure squared can be derived from the blood pressure waveform signal and the value for the PSV can be derived from the velocity-time signal. This feature is referred to by the label 'ABPmSquaredByPSV' in the detailed description.

By way of further example, in some embodiments, the first set of parameters may include a value for peak arterial blood pressure squared divided by mean arterial diameter. This feature is referred to by the label 'ABPpSquaredby-MeanDia' in the detailed description.

By way of further example, in some embodiments, the first set of parameters may include a value for the square of mean flow rate over a time interval. The mean flow rate can be computed from the velocity-time signal in combination with the arterial diameter signal.

In some embodiments, the one or more hemodynamic parameters output by the transfer function include one or more of: cardiac output, stroke volume, and stroke volume variation.

In some embodiments, the transfer function may be or comprise a machine learning model.

In some embodiments, the transfer function may be or comprise a (e.g. multiparametric) linear regression model.

Another aspect of the invention is a computer program product comprising a computer readable medium, the computer readable medium having computer readable code embodied therein, the computer readable code being configured such that, on execution by a suitable computer or processor, the computer or processor is caused to perform the method as set out above, or in accordance with any embodiment described in this document or any claim of this application.

The invention can also be embodied in hardware.

Accordingly, another aspect of the invention is a processing device comprising: an input/output; and one or more processors.

The one or more processors are adapted to perform the following steps:

receive ultrasound data from a blood vessel of the subject, wherein said ultrasound data includes B-Mode data, and pulsed wave Doppler data;
derive from the received ultrasound data a blood velocity-time signal representative of blood velocity at the measurement location of the blood vessel over a time window;
derive from the received ultrasound data an arterial diameter signal representative of a diameter of said at least one blood vessel, or a parameter proportional thereto, at the measurement location over the time window,
compute an ensemble average blood velocity waveform from a plurality of blood velocity waveforms present in the blood velocity-time signal, wherein each of the plurality of blood velocity waveforms corresponds to a single heartbeat;
determine a first set of parameters, including:

determining from the ensemble average blood velocity waveform one or more pre-defined blood velocity waveform features, and
determining from the arterial diameter signal one or more pre-defined arterial diameter waveform features;

retrieve a pre-defined transfer function configured to receive said first set of parameters as inputs and to generate the one or more hemodynamic parameters as output;
process the first set of parameters with the transfer function to derive values for the one or more hemodynamic parameters;
preferably generate a data output reflective of the one or more hemodynamic parameters.

Another aspect of the invention is a system comprising: the processing device described above, or in accordance with any embodiment or claim, an ultrasound acquisition apparatus comprising at least one transducer unit for acquiring ultrasound echo signal data of the at least one blood vessel of the subject, and a processing unit for processing the echo data to derive B-Mode data, and pulsed wave Doppler data. The input/output of the processing arrangement may be operatively coupled with an output of the ultrasound acquisition apparatus for receiving the B-Mode data, and the pulsed wave Doppler data.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;
Fig. 2 is a block diagram of an example processing device and system in accordance with one or more embodiments of the invention;
Fig. 3 shows a block diagram of a system for estimating hemodynamic parameters, according to one or more embodiments of the invention;
Fig. 4 shows a further block diagram of a system for estimating hemodynamic parameters, according to one or more embodiments of the invention, wherein the system includes modules for generating the transfer function;
Fig. 5 illustrates an example ensemble average blood velocity waveform, and illustrates a number of example waveform features which may be extracted from the ensemble average blood velocity waveform;

Fig. 6 illustrates a number of example velocity waveform features which may be extracted from a velocity-time signal in accordance with one or more embodiments;

Fig. 7 shows an example blood pressure waveform signal and an example velocity-time signal;

Figs. 8a-8d show a series of block diagrams illustrating different possible versions of a transfer function which may be used for deriving hemodynamic parameters, according to one or more embodiments of the invention;

Fig. 9 presents results of cardiac output values estimated using one version of the transfer function not in accordance with the invention, as compared against values computed using the Pulse Contour Cardiac Output (PiCCO) method;

Figs. 10-12 present results of cardiac output values estimated using three different versions of the transfer function in accordance with respective embodiments of the invention, as compared against values computed using the Pulse Contour Cardiac Output (PiCCO) method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for non-invasive hemodynamic monitoring of patients. The method comprises using ultrasound to obtain a continuous blood velocity signal and continuous arterial diameter signal. The method further comprises extracting waveform features from these two signals which can be used to estimate hemodynamic parameters. A transfer function is used to convert values of a pre-defined set of waveform features into values of one or more hemodynamic parameters. To enhance accuracy, ultrasound data is acquired over a time window and an ensemble average blood velocity waveform is computed from a series of waveforms detectable in the blood velocity-time signal.

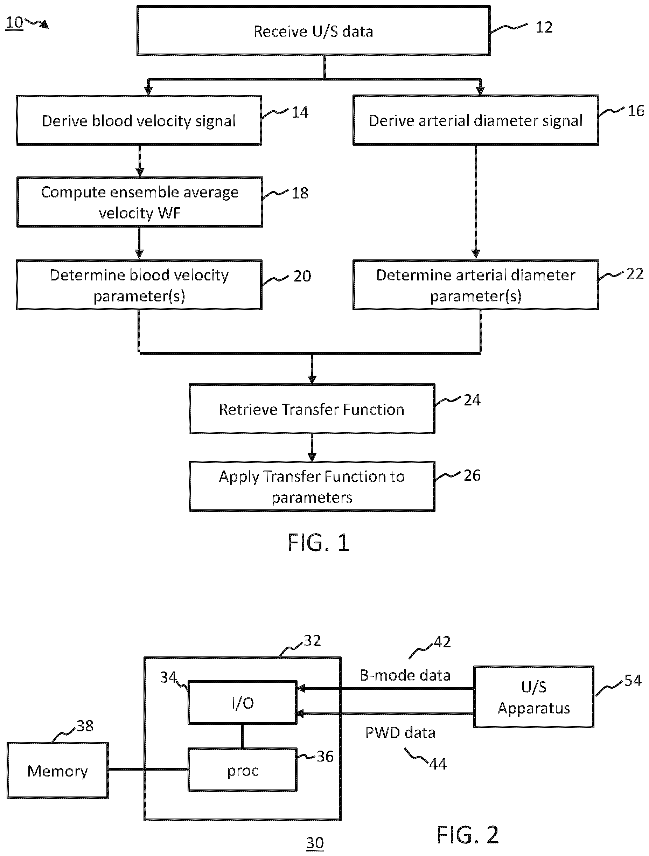

Fig. 1 outlines in block diagram form steps of an example method according to one or more embodiments. The steps will be recited in summary, before being explained further in the form of example embodiments.

The method 10 comprises receiving 12 ultrasound data from a blood vessel of the subject. This ultrasound data may include B-Mode data and pulsed wave Doppler data. The method 10 further comprises deriving 14 from the received ultrasound data a blood velocity-time signal representative of blood velocity at a measurement location of the blood vessel over a time window. The method 10 further comprises deriving 16 from the received ultrasound data an arterial diameter signal representative of a diameter of said at least one blood vessel, or a parameter proportional thereto, at the measurement location over the time window. The method further comprises computing 18 an ensemble average blood velocity waveform from a plurality of blood velocity waveforms present in the blood velocity-time signal. Each of the plurality of blood velocity waveforms may correspond to a single heartbeat. In other words, each of the plurality of blood velocity waveforms may represent blood velocity as a function of time over the course of a single heartbeat. In other words, each of the plurality of blood velocity waveforms may be a velocity waveform generated by a single heartbeat.

The method further comprises determining a first set of parameters, including: determining 20 from the ensemble average blood velocity waveform one or more pre-defined blood velocity waveform features; and determining 22 from the arterial diameter signal one or more pre-defined arterial diameter waveform features. The method further comprises retrieving 24 a pre-defined transfer function configured to receive said first set of parameters as inputs and to generate the one or more hemodynamic parameters as output. The method further comprises processing 26 the first set of parameters with the transfer function to derive values for the one or more hemodynamic parameters. The method may further comprise generating a data output reflective of the one or more hemodynamic parameters and for example transferring the data output to a further device such as a datastore or a computing device.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application.

In the illustrated example, the processing device further comprises an input/output 34 or communication interface.

In the illustrated example of Fig. 2, the system 30 further comprises an ultrasound acquisition apparatus 54. The ultrasound acquisition apparatus may comprise at least one transducer unit for acquiring ultrasound echo signal data of the at least one blood vessel of the subject. The ultrasound acquisition apparatus may further comprise a processing unit (not shown) for processing the echo data to derive B-Mode data 42, and pulsed wave Doppler data 44. The input/output 34 of the processing device may be operatively coupled with an output of the ultrasound acquisition apparatus 54 for receiving the B-Mode data 42, and the pulsed wave Doppler data 44.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

The one or more hemodynamic parameters output from the transfer function may include cardiac output, stroke volume, and/or stroke volume variation.

In some embodiments, the transfer function may be a machine learning model or a multiparametric linear regression model.

With regards to the one or more arterial diameter waveform features 22, these may for example comprise at least one of: a mean value of the arterial diameter over the time window, and/or a mean value of a cross-sectional area of the at least one blood vessel over the time window.

In some embodiments, the determining from the arterial diameter signal one or more pre-defined arterial diameter waveform features may comprise: computing an ensemble average arterial diameter waveform from a plurality of arterial diameter waveforms present in the arterial diameter signal; and computing from the ensemble average arterial diameter waveform an arterial diameter acceleration parameter indicative of a rise time between a foot of the ensemble average arterial diameter waveform and a peak of the ensemble average arterial diameter waveform.

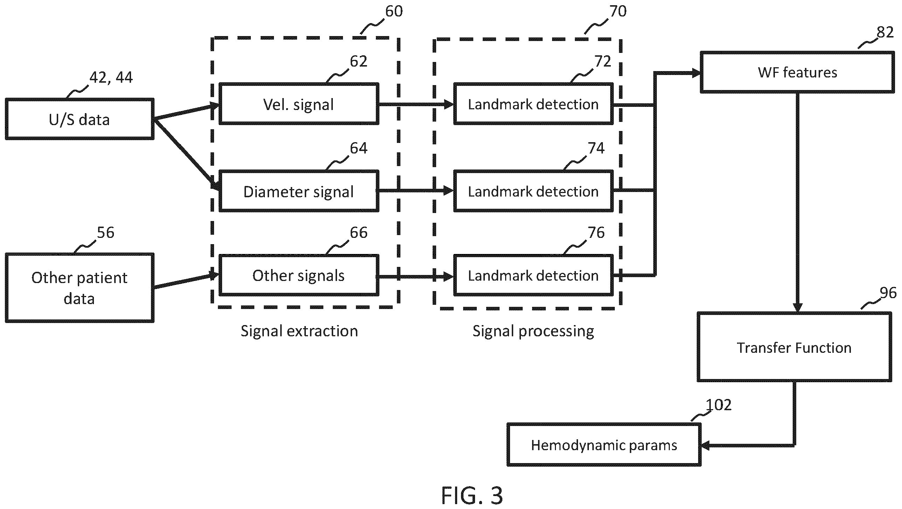

Fig. 3 shows a block diagram illustrating further an example processing flow which may be followed in accordance with one or more embodiments of the invention. The acquired ultrasound data, for example B-mode ultrasound data 42 and Pulsed Wave Doppler ultrasound data 44 provide inputs to a signal extraction procedure 60. The signal extraction procedure 60 comprises extracting a blood velocity-time signal 62 from the Pulsed Wave Doppler data and an arterial diameter signal 64 from the B-mode data. As will be explained in greater detail to follow, other patient data 56 may also be used as input to the method, such as blood pressure measurement data. From the other patient data, one or more further signals 66 may be derived, for example a blood pressure waveform signal.

Following signal extraction, a signal processing procedure 70 may be performed. The signal processing procedure 70 may comprise a process of landmark detection within the extracted signal. The purpose of landmark detection is to identify in the extracted signals 62, 64, 66 landmark points or fiducial points which can be used in extracting from the respective signals individual waveform sections, such as individual waveforms, and/or for use in extracting waveform features from the signals.

Fig. 3 shows a respective landmark detection process 72, 74, 76 performed for each of the blood velocity-time signal 62, the arterial diameter signal 64 and the optional one or more other signals 66. An example landmark detection process might involve for example detecting maxima and/or minima points in each respective signal, from which extraction of individual waveforms can be performed and/or direct extraction of one or more waveform features can be performed.

Following the landmark detection, waveform features 82 can be extracted, for example from individual waveforms separated using the landmark features or from the overall detected signal based on use of the landmark features.

For example, using the landmarks detected 72 in the velocity-time signal 62, a plurality of velocity waveforms present in the velocity-time signal can be extracted. For example, if the landmarks are maxima and minima, then this enables each waveform to be detected as the section of the velocity-time signal which includes one maximum point and one minimum point.

From the extracted waveforms, an ensemble average velocity waveform can be computed.

A set of one or more pre-defined velocity waveform features can be extracted from the ensemble average velocity waveform.

A set of pre-defined arterial diameter waveform features can further be extracted from the landmarked arterial diameter signal. For example, optionally a plurality of diameter waveforms may be extracted and one or more arterial diameter waveform features extracted. Alternatively, the arterial diameter waveform features may be extracted from the landmarked arterial diameter signal.

The extracted waveform features 82 form a first set of parameters which may then be input to the transfer function 96, wherein the transfer function is configured to receive said first set of parameters as inputs and to generate one or more hemodynamic parameters 102 as output.

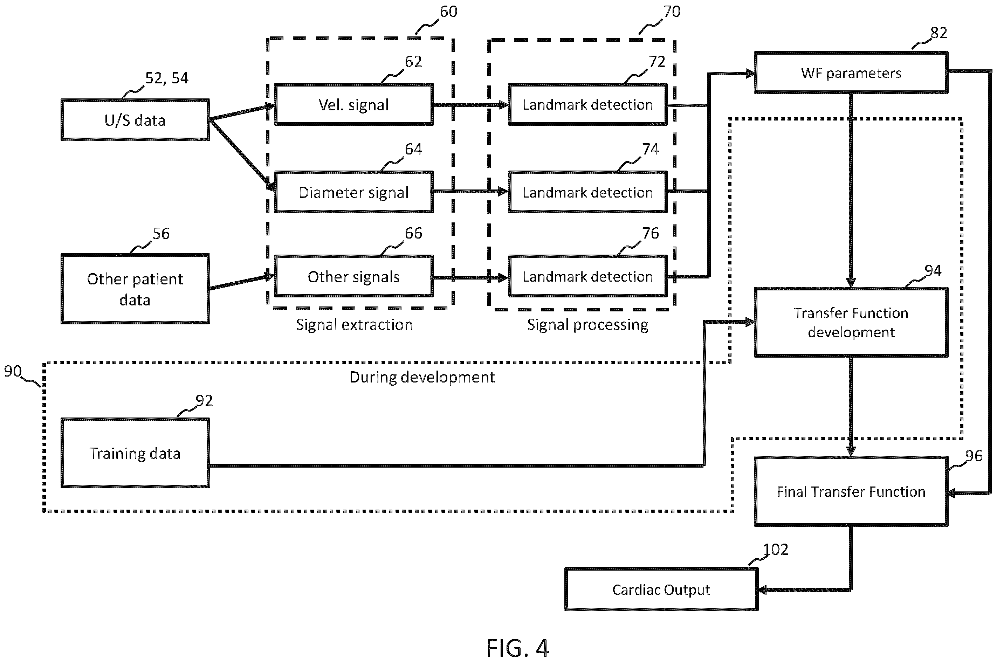

Fig. 4 shows the same processing flow as in Fig. 3 but with additional steps associated with development of the transfer function 96 shown in a transfer function development block 90. The transfer function development block 90 includes obtaining training data 92 for training or fitting of the transfer function. The training data is then used in a process of transfer function development 94. The transfer function development 94 may comprise for example training the transfer function using the training data. For example, the transfer function may be a machine learning function or model such as an artificial neural network. The transfer function may be a multiparametric regression model in further examples. More details regarding the training of the model will be outlined later.

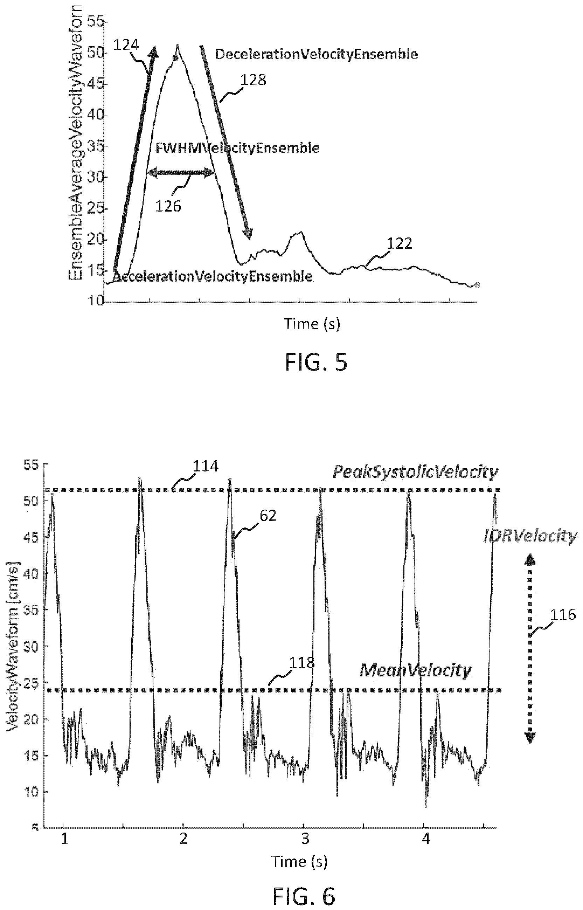

In Fig. 5, an example ensemble average blood velocity waveform 122 is illustrated. A number of example waveform features which may be extracted from the ensemble average velocity waveform are shown. These include an acceleration parameter 124 indicative of a steepness of a leading edge of the ensemble average blood velocity waveform. These further include a deceleration parameter 128 indicative of a steepness of a trailing edge of the ensemble average blood velocity waveform. These further include a velocity full-width-half-maximum (FWHM) parameter 126 indicative of a full-width-half-maximum of the ensemble average blood velocity waveform.

In addition to the waveform features derived from the ensemble average blood velocity waveform, the method may further involve computing from the blood velocity-time signal one or more additional pre-defined blood velocity waveform features. These additional parameters may provide further insights into the characteristics of the blood flow, potentially improving the accuracy of the hemodynamic parameter estimation. These further set of waveform features may be provided as additional inputs to the transfer function.

The one or more additional pre-defined blood velocity waveform features may comprise at least one of an interdecile range of the velocity-time signal over the time window, a mean value of blood velocity over the time window, a mean value of peak systolic velocity over the time window, a mean value of the blood velocity over the time window, normalized by a number of heart cycles spanned by the time window, and an integral of the velocity-time signal with respect to time over the time window, normalized by the number of heart cycles spanned by the time window.

Fig. 6 shows an example velocity-time signal 62 spanning a time window and which includes a plurality of velocity waveforms. Fig. 6 shows by way of illustration some of the example waveform features mentioned above which can optionally be extracted from the velocity-time signal alone. These may be included within an additional set of parameters which may form an additional input to the transfer function 96 in addition to the first set of parameters.

The example waveform features shown in Fig. 6 include peak systolic velocity 114, mean velocity 118, and velocity interdecile range (IDR) 116.

The peak systolic velocity 114 represents the maximum blood velocity reached during systole, while the mean velocity 118, indicated by a dashed line, represents the average blood velocity over the time window. The interdecile range of velocity 116, shown as the range between the 10th and 90th percentiles of the velocity values, provides a measure of the variability of the blood velocity.

In some embodiments, the method further comprises obtaining a blood pressure signal for the subject indicative of a blood pressure waveform for the subject over the time window. In some embodiments, the computing the first set of parameters further includes extracting from the blood pressure waveform signal one or more pre-defined blood pressure waveform features.

In some embodiments, the method further comprises computing from the blood pressure signal an ensemble average blood pressure waveform from a plurality of blood pressure waveforms present in the signal. In some embodiments, the determining the first set of parameters further includes deriving from the ensemble average blood pressure waveform one or more predefined blood pressure waveform features. Each of the plurality of blood pressure waveforms may correspond to a single heartbeat. In other words, each of the plurality of blood pressure waveforms may represent arterial blood pressure as a function of time over the course of a single heartbeat.

With regards to the one or more predefined blood pressure parameters, these may include a blood pressure acceleration parameter indicative of a rise time between a foot of the ensemble average blood pressure waveform and a peak of the ensemble average blood pressure waveform.

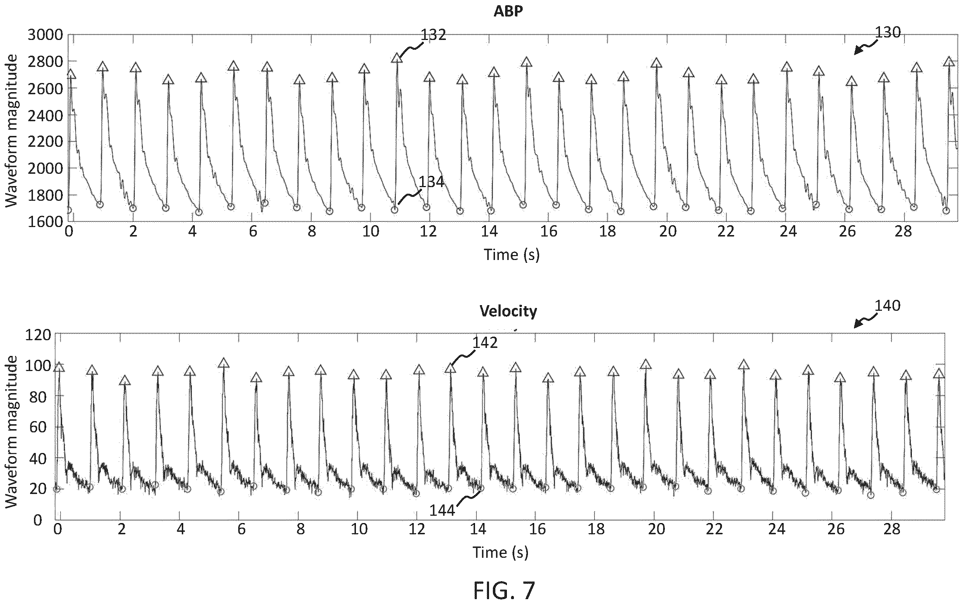

Fig. 7 shows a graphical representation of an arterial blood pressure (ABP) waveform signal 130 and a blood velocity-time signal 140.

To derive an ensemble average velocity waveform, blood pressure waveform, or diameter waveform, the method involves extracting multiple waveforms from the original relevant signal. This process begins by detecting fiducial points within the relevant signal (velocity-time signal, blood pressure signal, diameter signal) which correspond to specific, identifiable points in the cardiac cycle.

As shown in Fig. 7, fiducial detection involves identifying landmarks. For the ABP signal 130, this may comprise detecting maxima points, marked as ABP maxima 132 for arterial blood pressure and minimum points, marked as ABP minima 134 in Fig. 7. For the blood velocity signal 140, this may comprise detecting blood velocity maxima 142 and blood velocity minima 144. These points may serve as reference markers for aligning the individual waveforms.

Once the fiducial points are detected, the individual waveforms may be extracted and aligned based on these points. This alignment ensures that the waveforms are synchronized in time, allowing for accurate averaging. The aligned waveforms are then averaged together to create an ensemble average waveform. This ensemble average waveform represents a composite of the individual waveforms and provides a more stable and representative measure of the underlying hemodynamic signal.

The ensemble average waveform, whether it be for velocity, blood pressure, or diameter, is characterized by features that are less susceptible to the variability and noise present in individual waveforms. By averaging across multiple cardiac cycles, the method enhances the signal-to-noise ratio, allowing for more reliable extraction of waveform features.

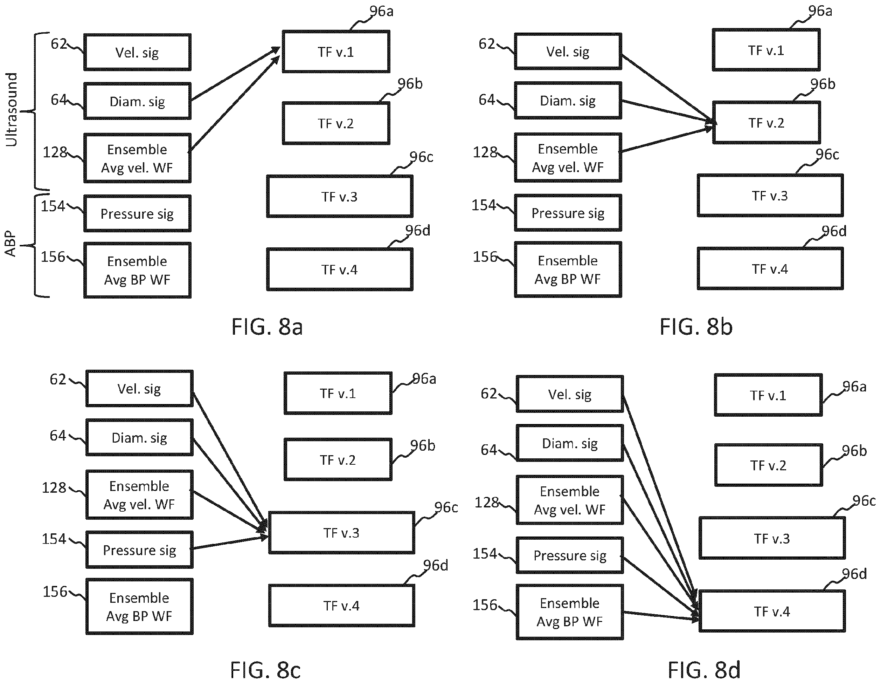

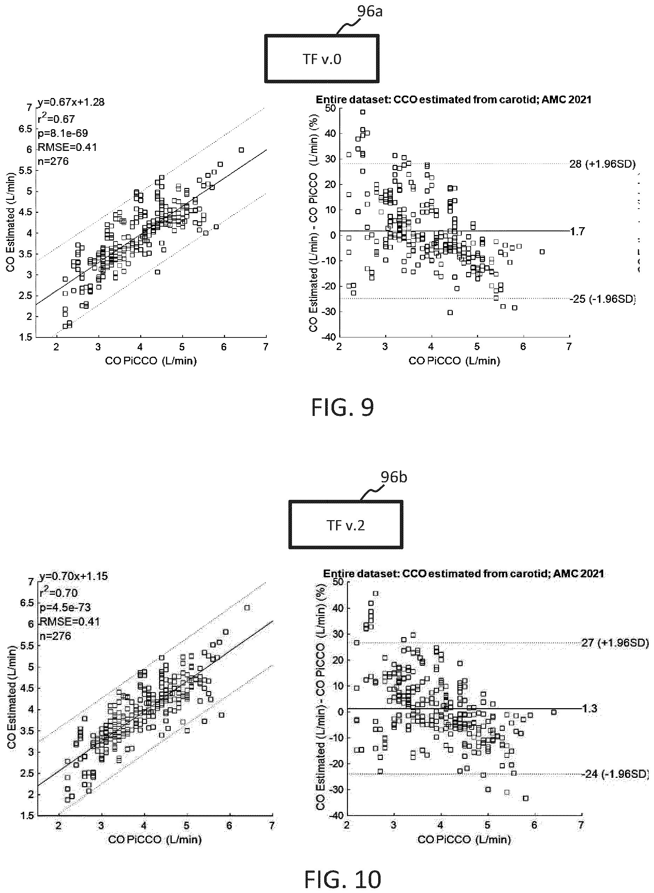

Referring to Figs. 8a-8d, a series of block diagrams are depicted that illustrate different versions of a transfer function (TF) used for deriving hemodynamic parameters. Each sub-figure (8a, 8b, 8c, and 8d) represents a different version of the transfer function, each designed to process a different subset of the possible signals and waveforms discussed above. The performance of each version will be discussed subsequently, with reference to Figs. 9-12.

It will be observed that every version of the transfer function includes use of waveform features extracted from an ensemble average velocity waveform 128 and from an arterial diameter signal 64. However the different transfer function versions employ different combinations of one or more of: waveform features extracted from a velocity-time signal 62; waveform features extracted from a blood pressure waveform signal 154; and waveform features extracted from an ensemble average blood pressure waveform signal 156. The blood pressure waveform signal and the ensemble average blood pressure waveform both derive from arterial blood pressure measurement data obtained from a blood pressure measurement means. The velocity-time signal 62, the arterial diameter signal 64 and the ensemble average velocity waveform 128 all derive from the acquired ultrasound data.

A first version of the transfer function (TF v. 1) 96a is shown in Fig. 8a. In this version, the inputs to the transfer function are only waveform features extracted from the ensemble average blood velocity waveform 128 and the arterial diameter signal 64.

A second version of the transfer function (TF v.2) 96b is shown in Fig. 8b. In this version, the inputs to the transfer function include waveform features extracted from the ensemble average blood velocity waveform 128, the arterial diameter signal 64 and from the velocity-time signal 62.

A third version of the transfer function (TF v.3) 96c is shown in Fig. 8c. In this version, the inputs to the transfer function include waveform features extracted from the ensemble average blood velocity waveform 128, the arterial diameter signal 64, the velocity-time signal 62, and from the blood pressure waveform signal 154.

A fourth version of the transfer function (TF v.4) 96d is shown in Fig. 8d. In this version, the inputs to the transfer function include waveform features extracted from the ensemble average blood velocity waveform 128, the arterial diameter signal 64, the velocity-time signal 62, the blood pressure waveform signal 154, and from the ensemble average blood pressure waveform signal 156.

With regards to the particular waveform features which are extracted from the various signals in the various versions of the transfer function, these will now be outlined in detail.

With regards to the waveform features which may be extracted from the ensemble average velocity waveform, and/or the other signals and waveforms which have been discussed above, the inventors have identified and tested a comprehensive set of waveform features which have been found to be good predictors or correlates of hemodynamic status. A list of these features will now be presented, along with the formulae for their calculation.

There will first be presented a list of example waveform features which may be extracted from the ensemble average velocity waveform, any one or more of which may be included among the first set of parameters:

*Table 1*

| Feature label | Description | Formula | Unit |
|---|---|---|---|
| AccelerationVelocityEnsemble | Acceleration of ensemble average velocity WF from diastole to PSV | $$\dfrac{PSV - EDV}{PSV(t) - EDV(t)}$$ | cm/s^2 |
| FWHMVelocityEnsemble | Time (full width) at half the max value of ensemble average velocity WF | - | s |
| RelFWHMVelocityEnsemble | Relative time of the FWHM over the cardiac cycle | proportion of the total pulse duration that the Full Width at Half Maximum (FWHM) occupies | No unit |
| RelAccelerationTimeVelocityEnsemble | Ratio of time to PSV to cardiac cycle duration | PSV(t)/ cardiac cycle length | No unit |

8

(continued)

| Feature label | Description | Formula | Unit |
|---|---|---|---|
| FW3QM_To_FWHMVelocityEnsemble | Ratio of full width at 75% of max value to ratio of full width at 50% of max value | | No unit |
| DecelerationVelocityEnsemble | Deceleration of velocity as measured from systolic peak (PSV) to subsequent foot (EDV) | $$\frac{PSV - EDV}{EDV(t) - PSV(t)}$$ | cm/s^2 |
| PSV = velocity at peak systolic velocity<br>EDV = velocity at end diastolic velocity<br>PSV(t) = time at peak systolic velocity<br>EDV(t) = time at end diastolic velocity<br>WF = "waveform" | | | |

As discussed above, in some embodiments, one or more waveform features may be extracted from an arterial blood pressure waveform signal and/or from an ensemble average arterial blood pressure waveform. There will now be outlined a set of example waveform features which may be extracted from an arterial blood pressure waveform signal and/or from an ensemble average arterial blood pressure waveform, any one or more of which may be utilized as part of the first set of parameters.

*Table 2*

| Feature label | Description | Formula | Unit |
|---|---|---|---|
| AccelerationABPEnsemble | Acceleration of ABP from diastole to systole | $$\frac{PSP - EDP}{PSP(t) - EDP(t)}$$ | mmHg/s |
| FWHMABPEnsemble | Full Width at half the max value of systolic pressure | - | s |
| RelFWHMABPEnsemble | Relative time of the FWHM over the total pulse width | - proportion of the total pulse duration that the Full Width at Half Maximum (FWHM) occupies | No unit |
| RelAccelerationTimeABPEnsemble | Ratio between time to systole (from diastole) to cardiac cycle duration | PSP(t) / total pulse width | No unit |
| FW3QM_To_FWHMABPEnsemble | Ratio of full width at 75% of Max to ratio of full width at 50% of max | | No unit |
| DecelerationABPEnsemble | Deceleration of ABP as measured from systole (PSP) to subsequent diastole (EDV) | $$\frac{PSP - PDP}{PDP(t) - PSP(t)}$$ | mmHg/s |
| SignalStabilityIndexABP | Signal stability index (SSI) of standardized ABP waveform | e.g. based on choosing the peak of the summated kernel density estimator | No unit |

(continued)

| Feature label | Description | Formula | Unit |
|---|---|---|---|
| SignalInstabilityIndexABP | Signal instability index of standardized ABP waveform | Inverse of SSI | No unit |
| ABP = arterial blood pressure<br>PSP = peak systolic pressure (value of the pressure signal at peak systole)<br>EDP = end diastolic pressure (value of the pressure signal and end diastole)<br>PSP(t) = time at peak systolic pressure<br>EDP(t) = time at end diastolic pressure | | | |

As mentioned above, in some embodiments, the first set of parameters may include waveform features extracted from an arterial diameter signal computed from the ultrasound data. There will now be outlined a list of example waveform features which may be extracted from an arterial diameter signal, any one or more of which may be included in the first set of parameters in accordance with one or more embodiments.

Table 3

| Feature label | Description | Formula | Unit |
|---|---|---|---|
| MeanDiameter | Average Diameter | Mean[d(i)] | cm |
| MeanCrossSectionalArea | Cross sectional area of diameter (average) | $$\frac{\pi * (Mean[d(i)])^2}{4}$$ | cm$^2$ |
| IDRDiameter | Interdecile range of diameter waveform | Percentile[d(i), 0.9] - Percentile[d(i), 0.1] | cm |
| SkewnessDiameter | skewness of diameter waveform | - | No unit |
| KurtosisDiameter | kurtosis of diameter waveform | - | No unit |
| SignalStabilityIndexDiameter | Signal instability index (SSI) of diameter waveform | e.g. based on choosing the peak of the summated kernel density estimator | No unit |
| SignalInstabilityIndexDiameter | Signal instability index of diameter pulse waveform | Inverse of Signal Stability Index (SSI) | No unit |
| d(i) = arterial diameter signal as a function of sample number, i, where samples are acquired at time intervals over the course of each heart cycle<br>Mean[d(i)] = mean blood vessel diameter over a single waveform<br>Percentile[d(i), 0.9] = 90$^{th}$ percentile of diameter signal, d(i)<br>Percentile[d(i), 0.1] = 10$^{th}$ percentile of diameter signal, d(i) | | | |

Signal Stability Index (SSI) measures the degree to which a signal remains consistent over time or across different samples. Higher values typically indicate a more stable or consistent signal. In a general case, a measure of SSI for any signal, including the blood pressure waveform signal, ensemble average blood pressure waveform and/or the diameter signal can be computed by segmenting a signal into multiple time windows and analyzing the variance or other statistical properties within and across these windows. Additionally or alternatively, computation of SSI may be based on choosing the peak of the summated kernel density estimator.

Signal Instability Index (SII) measures the degree to which a signal changes, fluctuates, or deviates from some norm over time. Higher values indicate more variability or instability. This can be understood as the inverse of SSI.

As mentioned above, in some embodiments, the first set of parameters may include waveform features extracted from a velocity time signal (e.g. a non ensemble-averaged velocity waveform). An example set of waveform features which might be extracted from a velocity-time signal will now be outlined, any one or more of which may be included among the first set of parameters.

*Table 4*

| Feature label | Description | Formula | Unit |
|---|---|---|---|
| Peak Systolic Velocity | Peak Systolic Velocity | Max[v(i)] for i=[0, velocity signal time window period] | cm/s |
| MeanVelocity | Mean of velocity signal | Mean[v(i)] | cm/s |
| SystolicFootVelocity | Systolic foot (or trough) | Min[v(i)] for i=[0... velocity signal time window period] | cm/s |
| IDRVelocity | Interdecile range of velocity-time signal | Percentile [v(i), 0.9] -Percentile [v(i), 0.1] | cm/s |
| VtiPerBeat | Area under the velocity-time signal (VTI) per heart beat | $\left( \left(\left(\sum_{i=1}^{n} v(i)\right)\right)/\text{Sample Freq}\right)/$ No of hearbeats | cm |
| MeanVelocityPerBeat | Mean velocity divided by the number of heartbeats in the epoch | Mean[v(i)]/ No of heartbeats | cm/s |
| Signal Stability Index Velocity | Signal stability index of standardized velocity waveform | | No unit |
| SignalnstabilityIndexVelocity | Signal instability index standardized velocity waveform | Inverse of SSI | No unit |
| MeanHR | Average heart rate in the epoch | no of heart beats / epoch duration | bpm |
| SqrtMeanHR | sqrt of mean HR | sqrt(mean HR) | bpm 1/2 |
| MeanVelocitySquare | Mean of velocity squared | Mean $[(v(i))]^2$ | $cm^2/s^2$ |
| SqrtPeakSystolicVelocity | Square root of PSV | sqrt(PSV) | $(cm/s)^{1/2}$ |
| SkewnessVelocity | skewness of velcoity waveform | - | No unit |
| KurtosisVelocity | kurtosis of velcoity waveform | - | No unit |
| PulsatilityIndexVelocity | Peak - systolic amplitude of velocity waveform divided by mean value of waveform | (PSV - EDV)/ Mean velocity | No unit |
| SDNN | The standard deviation of the NN intervals within a time-window. Note : NN interval is 'normal' RR intervals | std(RRi) | ms |
| RMSSD | The square root of the mean of the squares of successive differences between adjacent NN-intervals within a time-window. | rms (RRi) | ms |

v(i) = value of blood velocity as a function of sample number, i, where samples are acquired at time intervals

PSV = velocity at peak systolic velocity

EDV = velocity at end diastolic velocity

PSV(t) = time at peak systolic velocity

EDV(t) = time at end diastolic velocity

Percentile[d(i), 0.9] = 90th percentile of diameter signal, d(i)

Percentile[d(i), 0.1] = 10th percentile of diameter signal, d(i)

NN interval = the time period between successive normal cardiac R-wave peaks, and can be inferred from the systolic peak landmarks in the velocity-time signal

In some embodiments, the method may further comprise deriving a pressure-volume loop (PV loop) using an arterial blood pressure waveform and a blood volume waveform both spanning the same measurement time interval. A blood volume waveform can be computed from a blood velocity waveform by multiplying each velocity value by a value for arterial cross-sectional area, where arterial cross-sectional area can be estimated from a value derived for arterial diameter. If an arterial diameter signal exists spanning the same measurement time interval as for the pressure and velocity signals, then the time-varying diameter signal may be converted into a time-varying cross-sectional area signal, and then the cross-sectional area signal may be multiplied by the blood velocity signal to obtain a blood volume signal. As will be well known to the skilled person, a pressure-volume loop (or PV loop) can be used to approximate work done by the heart. In accordance with some embodiments of the invention, the first set of parameters may include one or more waveform features extracted from a PV loop computed for a cardiac cycle. The PV loop may optionally be computed using an ensemble average blood velocity waveform and ensemble average pressure waveform. Two examples of such waveform features are outlined below:

*Table 5*

| Feature label | Description | Formula | Unit |
|---|---|---|---|
| EnsemblePVLoopWorkDoneFeature | Area under the ensemble average PV loop as proxy for work done by the heart. | - | - |
| EnsemblePVLoopUpslopeFeature | Slope of PV Loop Upslope | tan [(Max[Vol]-Min[Vol]) / (Max[p] -Min[p])] | - |

Max[Vol] is the maximum volume value of the PV loop
Min[Vol] is the minimum volume value of the PV loop
Max[p] is the maximum pressure value of the PV loop
Min[p] is the minimum pressure value of the PV loop

In some embodiments, the first set of parameters may include one or more waveform features computed using a combination of the different waveforms and/or signals discussed above. Some examples will now be outlined below.

*Table 6*

| Feature label | Description | Formula | Unit |
|---|---|---|---|
| MeanFlowRate | Mean flow rate waveform | $$\frac{\pi}{4} \, mean(\, d(i)^2 * v(i)\, )$$ | cm³/s |
| MeanFlowRatePerBeat | Mean flow rate per beat | $$\frac{\pi}{4 * No\ of\ heart\ beats} \, mean(\, d(i)^2 * v(i)\, )$$ | cm³/s |
| MeanPeakFlowRate | Flow (cm^3/s) estimated with PSV x cross sectional area | $$\frac{\pi * AvgDia^2}{4} \, mean(PSV)$$ | cm³/s |
| MeanFlowRate Squared | Square of mean flow rate | square (MeanFlowRate) | cm^6/s^2 |
| DeltaVelbyDia | Square of difference between squared values of PSV and EDV divided by squared value of diameter squared | $$\frac{(PSV^2 - EDV^2)^2}{Diameter^2}$$ | cm 2/s4 |
| SqrtPSVByDiaSq | Square root of PSV times Diameter squared | $$\frac{sqrt(PSV)}{Diameter^2}$$ | 1/(cm*s ) |

(continued)

| Feature label | Description | Formula | Unit |
|---|---|---|---|
| ABPmSquaredByPSV | mean blood pressure squared divided by PSV | | (mmHg^2) / (cm/s) |
| ABPpSquaredbyMeanDia | pulse pressure squared divided by mean arterial Diameter | | (mmHg) / (cm^2) |

v(i) = value of blood velocity as a function of sample number, *i*, where samples are acquired at time intervals
d(i) = arterial diameter signal as a function of sample number, i, where samples are acquired at time intervals over the course of each heart cycle
PSV = velocity at peak systolic velocity
EDV = velocity at end diastolic velocity
PSV(t) = time at peak systolic velocity
EDV(t) = time at end diastolic velocity

Efficacy of the different versions of the transfer function (TF) for computing cardiac output discussed above with reference to Fig. 8a-8c will now be discussed, with reference to Fig. 9 to Fig. 12.

By way of providing a reference point, Fig. 9 (left) shows results of cardiac output (CO) computed using a simpler version of the transfer function which does not employ use of any waveform features derived from an ensemble average blood velocity waveform, but rather only waveform features extracted from the original velocity-time signal. This version of the transfer function is denoted as TF v.0 in Fig. 9. The values of CO as output by TF v.0 (y-axis) are plotted against the CO values computed for the same patient using the Pulse Contour Cardiac Output (PiCCO) method (x-axis). The PiCCO method represents the current gold standard in measurement of cardiac output. There is an r-squared coefficient of 0.67 and a p-value of 8.1e-69. This indicates a reasonably good correlation between the output CO values from the transfer function and the baseline values generated using the PiCCO method. Fig. 9 (right) shows the same data as Fig. 9 (left), except the y-axis represents the deltas (differences) between each estimated CO value output form the transfer function and the equivalent CO value determined using the PiCCO method. The lines marking upper and lower bounds of the 95% confidence interval are shown.

Fig. 10 (left) shows results of cardiac output (CO) computed using TF v.2 - see Fig. 8b. TF v.2 uses, as input, waveform features computed from the ensemble average velocity waveform 128, the velocity-time signal 62 and the arterial diameter signal 64. The values of CO as output by TF v.2 (y-axis) are plotted against the CO values computed for the same patient using the Pulse Contour Cardiac Output (PiCCO) method (x-axis). For this version of the TF, there is an r-squared coefficient of 0.70, which is higher than for the more simple TF v.0, and a p-value of 4.5e-73, which is lower than for TF v.0. This demonstrates that the inclusion of waveform features extracted from the ensemble average velocity waveform within the inputs to the TF improves the reliability of cardiac output values generated as output from the TF.

Fig. 10 (right) shows the same data as Fig. 10 (left), except that the y-axis represents the deltas (differences) between each estimated CO value output form the transfer function and the equivalent CO value determined using the PiCCO method. The lines marking upper and lower bounds of the 95% confidence interval are shown.

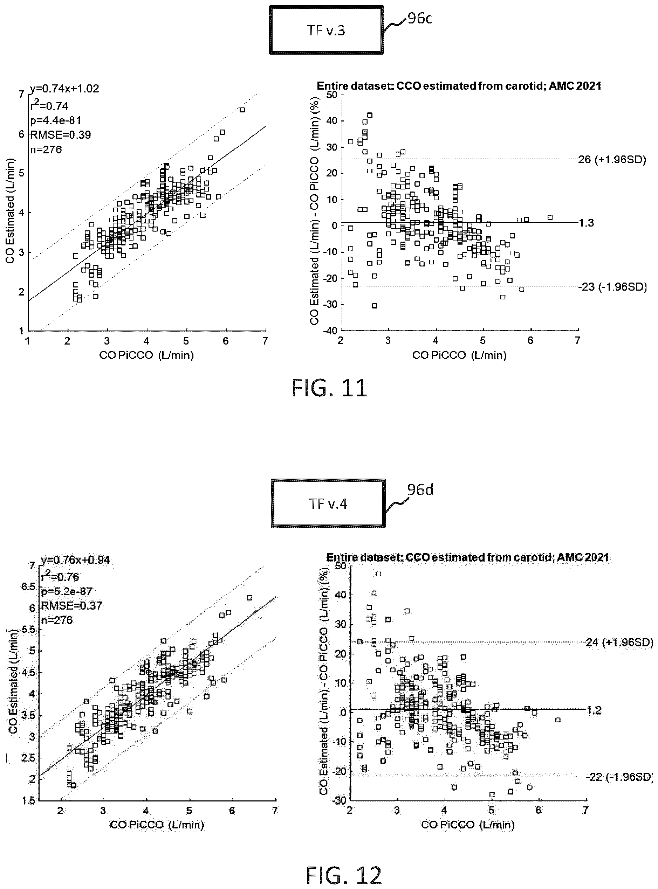

Fig. 11 shows the same two graphs as for Fig. 10, but presents the data for the version of the transfer function TF v.3 - see Fig. 8c. There is an r-squared coefficient between the CO values predicted by TF v.3 and equivalent values measured using the PiCCO method of 0.74, which is higher than for the TF v.2 and v.0, and a p-value of 4.4e-81, which is lower than for TF v.2 and v.0. This demonstrates that the additional inclusion of waveform features extracted from the blood pressure waveform signal 154 within the inputs to the TF improves the reliability of cardiac output values generated as output from the TF.

Fig. 12 shows the same two graphs as for Fig. 10 and Fig. 11, but presents the data for the version of the transfer function TF v.4 - see Fig. 8d. There is an r-squared coefficient between the CO values predicted by TF v.4 and equivalent values measured using the PiCCO method of 0.76, which is higher than for the TF v.3, TF v.2 and v.0, and a p-value of 5.2e-87, which is lower than for TF v.3, TF v.2 and v.0. This demonstrates that the additional inclusion of waveform features extracted from the blood pressure waveform signal 154 within the inputs to the TF improves the reliability of cardiac output values generated as output from the TF.

In some embodiments, a transfer function may be employed which is trained to utilise all of the different waveform features outlined in the Tables above. In order words, all of the different waveform features may form inputs to the transfer function. This approach allows for a comprehensive analysis of the subject's hemodynamic status, potentially improving the accuracy and reliability of the estimation.

As discussed above, embodiments of the invention involve computation of an ensemble average blood velocity waveform from a plurality of blood velocity waveforms extracted from the blood velocity-time signal, wherein each of the plurality of blood velocity waveforms corresponds to blood velocity over a single heartbeat. This process involves the alignment and averaging of the multiple blood velocity waveforms corresponding to multiple heartbeats to produce a single waveform that represents the typical pattern of blood flow velocity over a single heartbeat (a single cardiac cycle).

To compute the ensemble average blood velocity waveform, individual blood velocity waveforms for individual heartbeats are first identified within the blood velocity-time signal. Each waveform corresponds to a single cardiac cycle and is characterized by a systolic peak followed by a diastolic trough. The waveforms are then aligned with respect to a common feature, such as the onset of systole or the peak systolic velocity, to ensure that the corresponding phases of the cardiac cycle are synchronized across all waveforms.

Once aligned, the waveforms are averaged together to produce the ensemble average waveform. Mathematically, this can be expressed as follows:

$$V_{\text{ensemble}}(t) = \frac{1}{N} \sum_{i=1}^{N} V_i(t)$$

where $V_{\text{ensemble}}(t)$ is the ensemble average blood velocity waveform, $N$ is the number of blood velocity waveforms included in the average, $V_i(t)$ is the blood velocity at time $t$ for the i-th waveform, and the summation is performed over the aligned time points t of the cardiac cycle.

The resulting ensemble average waveform provides a stable and representative depiction of the blood velocity waveform, which can be used to compute various waveform features with reduced variability due to physiological fluctuations or measurement noise. For example, the ensemble average waveform can be used to determine the acceleration parameter, which reflects the steepness of the leading edge of the waveform, or the velocity full-width-half-maximum (FWHM) parameter, which provides a measure of the dispersion of the blood velocity around the systolic peak, and/or any combination of one or more of the other waveform features discussed above.

In some cases, the ensemble averaging process may be weighted to account for variations in the quality or reliability of individual waveforms. For instance, waveforms that are less noisy or that have clearer landmark features may be given greater weight in the averaging process. This can be achieved by introducing a set of weights $w_i$ such that:

$$V_{\text{ensemble}}(t) = \frac{\sum_{i=1}^{N} w_i V_i(t)}{\sum_{i=1}^{N} w_i}$$

where $w_i$ is the weight assigned to the *i*-th waveform, and the weights are normalized by their sum to ensure that the ensemble average waveform remains on the same scale as the individual waveforms.

The same process outlined above can also be applied for computing an ensemble average blood pressure waveform and/or an ensemble average arterial diameter waveform according to one or more embodiments.

Embodiments of the invention make use of a pre-defined transfer function to convert the first set of parameters or the second set of parameters into values for one or more hemodynamic parameters. There has already been discussed above different options for the set of inputs to the transfer function. There will now be outlined details and options relating to the training and architecture of suitable transfer functions according to various embodiments of the invention.

In some embodiments, the retrieval of a pre-defined transfer function is a process that involves accessing a stored model that has been previously developed and validated to estimate hemodynamic parameters based on a set of input features. The transfer function may be stored in a database or a library within the processing device 32 or may be accessed remotely via a network connection. The transfer function is pre-defined in the sense that it has been established prior to its use in the method and does not require real-time development or training during the estimation process.

The configuration of the transfer function to receive the first/second set of parameters as inputs involves setting up the function to accept the specific types of data that have been computed from the ultrasound measurements. This may include defining the input structure, such as the order and format of the parameters, to match the expected input format of the transfer function. For example, the transfer function may require inputs to be provided as a vector or matrix of numerical values, where each element corresponds to a specific parameter, such as the acceleration parameter, deceleration parameter, or velocity FWHM parameter.

Once the first set of parameters is input into the transfer function, the function applies a series of mathematical operations to generate the output hemodynamic parameters. These operations may include linear or non-linear transformations, statistical analysis, and/or application of machine learning algorithms. The specific operations depend on the nature of the transfer function; for instance, a multiparametric linear regression model would apply a linear combination of the input parameters weighted by regression coefficients, while a machine learning model might use a more complex algorithm such as a neural network to process the inputs.

In one set of embodiments, the transfer function comprises a multiparametric regression model or function.

By way of example, a transfer function making use of a multiparametric linear regression model to estimate a hemodynamic parameter, such as cardiac output (CO), might employ a general mathematical function of the form expressed below:

$$CO = \beta_0 + \beta_1 \cdot p_1 + \beta_2 \cdot p_2 + \beta_3 \cdot p_3 + \cdots + \beta_n \cdot P_n$$

where $\beta_0$ is the intercept term, $\beta_1$ to $\beta_n$ are the regression coefficients for each input parameter, and $p_1$ to $p_n$ represent the individual parameters, values of which form the first set of parameters.

Of course, cardiac output (CO) is just one example of a hemodynamic parameter which a linear regression function could be trained or fitted to derive. The transfer function might comprise a linear regression equation which is configured to output a different hemodynamic parameter. In some embodiments, the transfer function may comprise a plurality of linear regression equations, each configured to output a different respective hemodynamic parameter. The plurality of linear regression equations might be configured to receive and process values for the same set of input parameters (by applying different respective sets of coefficients to these parameters) or the plurality of linear regression equations could be configured to receive as inputs different combinations of one or more of the parameters. In some embodiments, the transfer function could comprise a system of linear regression equations, each configured to process a same set of parameters (e.g. a same set of waveform features), and each associated with a different respective set of coefficients for applying to the input waveform features, wherein applying the transfer function comprises applying the multiple sets of coefficients to a common set of values of a set of parameters, to thereby generate as output values for a plurality of hemodynamic parameters. For example, the sets of coefficients could be represented in a two-dimensional matrix and the set of values for the input parameters (e.g. waveform features) could be represented in a one-dimensional matrix, wherein the two-dimensional matrix and the one-dimensional matrix are multiplied together.

By way of a particular example, presented for purposes of illustration, an example regression function might make use of the following function:

$$CO = \beta_0 + \beta_1 \cdot V_{\mathrm{acc}} + \beta_2 \cdot V_{\mathrm{dec}} + \beta_3 \cdot V_{\mathrm{FWHM}}$$

where $\beta_0$ is the intercept term,
$\beta_1$ to $\beta_n$ are the regression coefficients for each input parameter,
$V_{\mathrm{acc}}$ is the ensemble velocity acceleration parameter "AccelerationVelocityEnsemble",
$V_{\mathrm{dec}}$ is the ensemble velocity deceleration parameter "DecelerationVelocityEnsemble", and
$V_{\mathrm{FWHM}}$ is the ensemble velocity FWHM parameter, "FWHMVelocityEnsemble".

Thus, for a multiparametric linear regression model, the transfer function is typically a linear equation that relates the input parameters to the output hemodynamic parameters through a set of regression coefficients. These coefficients are determined by fitting the model to a training dataset using least squares regression or another fitting technique.

The process of training a multiparametric linear regression function comprises a process of fitting the coefficients of the multiparametric linear regression equation to a training dataset. This may comprise for example a process of minimising a loss function, where the loss function is a function of the residual between prediction output values from the linear regression function and the actual data values. A typical loss function might be for example be a mean square error function of the residual. The coefficients of the regression function are iteratively adjusted to minimise the value of the loss function.

In some embodiments, a stepwise multiparametric regression fitting process may be performed to fit/train the transfer function.

In some embodiments, the transfer function training may be performed using a k-fold approach. In a k-fold approach, the training dataset is split into *k* smaller, non-overlapping subsets (or folds). Subsequently, each of the *k* subsets is used in a separate respective training iteration used to train or fit the coefficients of the linear regression function. At the end of this procedure, there are *k* versions of the linear regression function coefficient set. These can then be averaged together to form a final set of regression function coefficients.

Instead of a multiparametric regression function, a different type of machine learning model such as a neural network might be used as the transfer function.

In the case of a machine learning model, such as a neural network, the process is more complex. The input parameters are fed into the network, which consists of multiple layers of interconnected nodes or neurons. Each neuron applies a non-linear activation function to the weighted sum of its inputs and passes the result to the next layer. The final layer produces the output, which is the estimated hemodynamic parameter. The weights of the connections between neurons are adjusted during the training phase to minimize the error between the estimated and actual hemodynamic parameters.

The application of the transfer function is not limited to a single hemodynamic parameter. In practice, the transfer function may be designed to estimate multiple parameters simultaneously. For example, a single transfer function could output an array of hemodynamic parameters such as cardiac output, stroke volume, and stroke volume variation, each estimated from the same set of input parameters.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

**1.** A computer implemented method (60) for deriving one or more hemodynamic parameters of a subject, comprising:

receiving (12) ultrasound data from a blood vessel of the subject, wherein said ultrasound data includes B-Mode data, and pulsed wave Doppler data;
deriving (14) from the received ultrasound data a blood velocity-time signal representative of blood velocity at a measurement location of the blood vessel over a time window;
deriving (16) from the received ultrasound data an arterial diameter signal representative of a diameter of said at least one blood vessel, or a parameter proportional thereto, at the measurement location over the time window, computing (18) an ensemble average blood velocity waveform from a plurality of blood velocity waveforms present in the blood velocity-time signal, wherein each of the plurality of blood velocity waveforms corresponds to a single heartbeat;
determining a first set of parameters, including:

determining (20) from the ensemble average blood velocity waveform one or more pre-defined blood velocity waveform features, and

determining (22) from the arterial diameter signal one or more pre-defined arterial diameter waveform features;

retrieving (24) a pre-defined transfer function configured to receive said first set of parameters as inputs and to generate the one or more hemodynamic parameters as output;
processing (26) the first set of parameters with the transfer function to derive values for the one or more hemodynamic parameters.

2. The method of claim 1,
wherein the one or more pre-defined blood velocity waveform features determined from the ensemble average blood velocity waveform include: an acceleration parameter indicative of a steepness of a leading edge of the ensemble average blood velocity waveform.

3. The method of claim 1 or 2,
wherein the one or more pre-defined blood velocity waveform features determined from the ensemble average blood velocity waveform include: a deceleration parameter indicative of a steepness of a trailing edge of the ensemble average blood velocity waveform.

4. The method of any preceding claim,
wherein the one or more pre-defined blood velocity waveform features determined from the ensemble average blood velocity waveform include: a velocity full-width-half-maximum (FWHM) parameter indicative of a full-width-half-maximum of the ensemble average blood velocity waveform.

5. The method of any preceding claim,

wherein the method further comprises obtaining a blood pressure waveform signal for the subject indicative of a blood pressure waveform for the subject over the time window; and
wherein the computing the first set of parameters further includes: extracting from the blood pressure waveform signal one or more pre-defined blood pressure waveform features.

6. The method of claim 5, wherein the method further comprises:

computing from the blood pressure waveform signal an ensemble average blood pressure waveform from a plurality of blood pressure waveforms present in the blood pressure signal; and
wherein the determining the first set of parameters further includes deriving from the ensemble average blood pressure waveform one or more predefined blood pressure waveform features.

7. The method of claim 6, wherein the one or more predefined blood pressure waveform features include a blood pressure acceleration parameter indicative of a rise time between a foot of the ensemble average blood pressure waveform and a peak of the ensemble average blood pressure waveform.

8. The method of any preceding claim, wherein the computing the first set of parameters further includes: computing from the blood velocity-time signal one or more further pre-defined blood velocity waveform features.

9. The method of any claim 8, wherein the one or more further pre-defined blood velocity waveform features comprise at least one of:

an interdecile range of the velocity-time signal over the time window;
a mean value of blood velocity over the time window;
a mean value of peak systolic velocity over the time window;
a mean value of the blood velocity over the time window, normalized by a number of heart cycles spanned by the time window;
an integral of the velocity-time signal with respect to time over the time window, normalized by the number of heart cycles spanned by the time window.

10. The method of any of claims 1-9, wherein the one or more arterial diameter waveform features comprise at least one of:

a mean value of the arterial diameter over the time window;
a mean value of a cross-sectional area of the at least one blood vessel over the time window.

11. The method of any of claims 1-10, wherein the computing from the arterial diameter signal one or more pre-defined arterial diameter waveform features comprises:

computing an ensemble average arterial diameter waveform from a plurality of arterial diameter waveforms present in the arterial diameter signal; and
computing from the ensemble average arterial diameter waveform an arterial diameter acceleration parameter indicative of a rise time between a foot of the ensemble average arterial diameter pulse waveform and a peak of the ensemble average arterial diameter pulse waveform.

12. The method of any of claims 1-11, wherein:

the transfer function is a machine learning model; and or
the transfer function is a multiparametric linear regression model.

13. A computer program product comprising a computer readable medium, the computer readable medium having computer readable code embodied therein, the computer readable code being configured such that, on execution by a computer or processor, the computer or processor is caused to perform the method as claimed in any of claims 1-12.

14. A processing device (32) comprising:

an input/output (34); and
one or more processors (36), adapted to:

receive (12) ultrasound data from a blood vessel of the subject, wherein said ultrasound data includes B-Mode data, and pulsed wave Doppler data;
derive (14) from the received ultrasound data a blood velocity-time signal representative of blood velocity at the measurement location of the blood vessel over a time window;
derive (16) from the received ultrasound data an arterial diameter signal representative of a diameter of said at least one blood vessel, or a parameter proportional thereto, at the measurement location over the time window,
compute (18) an ensemble average blood velocity waveform from a plurality of blood velocity waveforms present in the blood velocity-time signal, wherein each of the plurality of blood velocity waveforms corresponds to a single heartbeat;
determine a first set of parameters, including:

determining (20) from the ensemble average blood velocity waveform one or more pre-defined blood velocity waveform features, and
determining (22) from the arterial diameter signal one or more pre-defined arterial diameter waveform features;

retrieve (24) a pre-defined transfer function configured to receive said first set of parameters as inputs and to generate the one or more hemodynamic parameters as output;

process (26) the first set of parameters with the transfer function to derive values for the one or more hemodynamic parameters;
preferably generate a data output reflective of the one or more hemodynamic parameters.

15. A system (30) comprising:

the processing device (32) of claim 14; and
an ultrasound acquisition apparatus (54) comprising at least one transducer unit for acquiring ultrasound echo signal data of the at least one blood vessel of the subject, and a processing unit for processing the echo data to derive B-Mode data (42), and pulsed wave Doppler data (44);
wherein the input/output (34) of the processing arrangement is operatively coupled with an output of the ultrasound acquisition apparatus for receiving the B-Mode data, and the pulsed wave Doppler data.

10 ↘

| Receive U/S data | 12 |

| Derive blood velocity signal | 14 |    | Derive arterial diameter signal | 16 |

| Compute ensemble average velocity WF | 18 |

| Determine blood velocity parameter(s) | 20 |    | Determine arterial diameter parameter(s) | 22 |

| Retrieve Transfer Function | 24 |

| Apply Transfer Function to parameters | 26 |

FIG. 1

32

42

34

I/O

B-mode data

U/S Apparatus    54

38

Memory

36

proc

PWD data

44

30

FIG. 2

WF features — 82

Transfer Function — 96

Hemodynamic params — 102

Signal processing — 70

Landmark detection — 72

Landmark detection — 74

Landmark detection — 76

Signal extraction — 60

Vel. signal — 62

Diameter signal — 64

Other signals — 66

U/S data — 42, 44

Other patient data — 56

FIG. 3

FIG. 4

FIG. 5

FIG. 6

FIG. 7

FIG. 8a

FIG. 8b

FIG. 8c

FIG. 8d

EP 4 721 674 A1

96a

TF v.0

FIG. 9

96b

TF v.2

FIG. 10

TF v.3 — 96c

y=0.74x+1.02
$r^2$=0.74
p=4.4e-81
RMSE=0.39
n=276

CO Estimated (L/min) — CO PiCCO (L/min)

Entire dataset: CCO estimated from carotid; AMC 2021

26 (+1.96SD)

1.3

-23 (-1.96SD)

CO PiCCO (L/min)

**FIG. 11**

TF v.4 — 96d

y=0.76x+0.94
$r^2$=0.76
p=5.2e-87
RMSE=0.37
n=276

CO Estimated (L/min)

Entire dataset: CCO estimated from carotid; AMC 2021

24 (+1.96SD)

1.2

-22 (-1.96SD)

CO PiCCO (L/min)

**FIG. 12**

Europäisches
Patentamt

European
Patent Office

Office européen
des brevets

# EUROPEAN SEARCH REPORT

**Application Number**

EP 24 20 4126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 378 394 A1 (KONINKLIJKE PHILIPS NV [NL]) 5 June 2024 (2024-06-05) * paragraphs [0014] - [0044], [0119] - [0194]; claims; figures * | 1-15 | INV. A61B8/04 A61B8/06 A61B8/08 A61B8/00 |
| A | US 2023/210491 A1 (JOSHI ROHAN [NL]) 6 July 2023 (2023-07-06) * paragraphs [0013] - [0048]; claims; figures * | 1-15 | ADD. A61B8/02 |
| A | TIMOTHY IRVINE ET AL: "A digital 3-dimensional method for computing great artery flows: In vitro validation studies", JOURNAL OF THE AMERICAN SOCIETY OF ECHOCARDIOGRAPHY., vol. 13, no. 9, 1 September 2000 (2000-09-01), pages 841-848, XP055705599, US ISSN: 0894-7317, DOI: 10.1067/mje.2000.106049 * Introduction; Methods * | 1-15 | |

TECHNICAL FIELDS SEARCHED (IPC)

A61B

The present search report has been drawn up for all claims

| Place of search | Date of completion of the search | Examiner |
|---|---|---|
| Munich | 19 February 2025 | Mundakapadam, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 4126

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2025

| Patent document cited in search report | | | Publication date | Patent family member(s) | | Publication date |
|---|---|---|---|---|---|---|
| EP 4378394 | A1 | | 05-06-2024 | NONE | | |
| US 2023210491 | A1 | | 06-07-2023 | CN | 115715170 A | 24-02-2023 |
| | | | | EP | 4164501 A2 | 19-04-2023 |
| | | | | JP | 2023528679 A | 05-07-2023 |
| | | | | US | 2023210491 A1 | 06-07-2023 |
| | | | | WO | 2021250234 A2 | 16-12-2021 |

EPO FORM P0459

For more details about this annex : see Official Journal of the European Patent Office, No. 12/82